# EUROPEAN PATENT SPECIFICATION

(11) **EP 0 453 419 B1**
(45) Date of publication and mention of the grant of the patent: **04.09.1996**
(21) Application number: 91830153.2
(22) Date of filing: 17.04.1991
(51) Int. Cl.: G01N 33/569, C07K 2/00

(54) **Use of the SP-2 monoclonal antibody for diagnostics and for monitoring of the HIV infection progress**
Benutzung von SP-2 monoklonalen Antikörpern zur Diagnostik und zur Beobachtung des HIV-Infektionsfortschritts
Utilisation d'anticorps monoclonal SP-2 pour le diagnostic et le contrôle de la progression de l'infection HIV

(30) Priority: 23.04.1990 IT 2011390
(43) Date of publication of application: 23.10.1991
(73) Proprietor: CONSIGLIO NAZIONALE DELLE RICERCHE, I-00185 Roma (IT); UNIVERSITA' DEGLI STUDI "G. D'ANNUNZIO", I-66100 Chieti (IT)
(72) Inventor: Iacobelli, Stefano, I-00198 Rome (IT)
(74) Representative: de Simone, Domenico

(56) References cited:
- BREAST CANCER RESEARCH AND TREATMENT. vol. 11, no. 1, November 1988, BOSTON MA USA pages 19 - 30; S. IACOBELLI ET AL.: 'Measurement of a breastcancer associated antigen detected by monoclonal antibody SP-2 in sera ofcancer patients.'
- THE JOUNAL OF INFECTIOUS DISEASES, vol. 164, no. 4, October 1991, Chicago, Illinois (US); S. IACOBELLI et al.; p. 819

## Description

This invention relates to the use of SP-2 monoclonal antibody for the clinical diagnostics and for the monitoring of the progress of the HIV (human immunodeficiency virus) infection as well as in the production of kits for carrying out said diagnostic and monitoring analysis.

It is well known that the SP-2 monoclonal antibody (IgG1 subclass) reacts with an antigen of 90,000 dalton molecular weight, not yet fully characterized, designated as 90K, said antigen being found in the tumoral tissue and in the sera of patients suffering from breast cancer and other neoplastic diseases (S. Iacobelli et al.; Cancer Research 46, 3005-10, 1986; Anticancer Research 8, 761-4, 1988; Breast Cancer Research and Treatment 11, 19-30, 1988; Gynecologic Oncology, 35, 286-9, 1989).

Now it has been surprisingly found that the antigen 90K is also present in the sera of patients infected with HIV and that the positivity percentage and the antigen average concentrations in serum are proportional to the extent of the infection, with increasing values on passing from the HIV-seropositivity conditions in asymptomatic subjects to the ARC (AIDS related complex) and to the AIDS (acquired immunodeficiency syndrome). Furthermore 90K levels higher than those in normal subjects are found in the serum of anti-HIV seronegative subjects at high risk of HIV infection. The assay of the 90K antigen is performed by utilizing the monoclonal antibody SP-2, purified from the correspondent hybridoma cell line, deposited at C.N.C.M. N.I-1083, as previously described by the author of the present invention, Iacobelli et al., 1986, Cancer Research, 46, 3005-3010 and Iacobelli et al., 1988, Breast Cancer Research and Treatment, 11, 19-30. The assay thus represents a very useful support for the early diagnosis of the HIV infection just before the patient shows the symptoms of the disease and for the monitoring of the progress of the infection itself. Accordingly, the test can be employed for selecting the patients who are to be treated therapeutically, and then for evaluating the efficacy of the treatment itself.

Thus it is an object of the present invention the use of the SP-2 monoclonal antibody for an in vitro assay of the 90K antigen from biological fluids or tissues useful for the clinical diagnostics related to HIV infection.

Preferably said in vitro assay of the 90K antigen is performed to make a diagnosis of the disease stage during the HIV infection, to foresee the evolution of the infection through ARC or AIDS in anti-HIV seropositive asymptomatic subjects, to monitor the progress of HIV infection, to evaluate the efficacy of a specific (antiviral) or of aspecif therapy, to early point out the serum-conversion of anti-HIV seronegative subjects at risk.

According to the present invention said in vitro assay is performed through immunoassays, preferably comprised within the group of assays utilizing as revealing systems, enzymatic, radioisotopic or chemioluminescent systems. Said enzymatic systems comprise ELISA or EIA assays, said radio isotopic systems comprise IRMA or RIA systems.

It is a further object of the present invention the use of the monoclonal antibody SP-2 for the preparation of diagnostic kits to perform said in vitro assays.

The present invention will be described with refer to the following example:

### Example 1

Kit for the clinical diagnostics and the monitoring of the progress of the HIV infection
- small polystyrene beads sensitized with SP-2 mouse monoclonal IgG No. 100
- a tracer consisting of SP-2 mouse monoclonal IgGs Iabeled with ¹²⁵I, buffer, proteins, stabilizing and preserving agents;
   maximum radioactivity = 496 kBq (13.4 µCi) at the calibration date 21 ml
- standard 0 made up of a buffer, proteins and preserving agents 30 ml
- standards 1-4 containing the antigen 90K at the respective concentrations of 5-10-20-40 U/ml, HBsAg- and HIV-1-negative human serum, buffer, proteins and preserving agents 1 ml

The material mentioned above has been employed for analysing blood samples coming from the following subjects:
- 65 control subjects (normal subjects)
- 80 seronegative homosexuals exposed to the risk of AIDS
- 72 asymptomatic seropositive subjects
- 39 subjects suffering from the AIDS-Related Complex (ARC),
- 41 subjects suffering from AIDS.

The blood samples were subjected to centrifugation and the sera were separated and stored at -30°C.

At the moment of the analysis, the samples were thawed and diluted 1/100 with the "zero" standard (10 µl of the sample + 990 µl of the zero standard) and all reactants had been conditioned at room temperature (20-25°C). Employing 20-well plastic reaction plates, the wells were identified for carrying out the assay of the standard and of the samples at least in duplicate. The standard 0 (100 µl) was distributed in all wells. The standards 0-4 (100 µl) and samples diluted (100 µl) were distributed in the respective wells. A small bead was put into each well. The wells were covered with a self-adhesive paper for preventing the sample from evaporating. The reaction plates were stirred mildly in order to remove any possible air bubbles, and keeping carefully said small beads completely covered.

The reaction plates were incubated for one hour at 37°C. At the end of the incubation, the self-adhesive paper was removed and the liquid was aspirated. Each well and the small bead contained in the same were repeatedly washed with a total volume of 15 ml of deionized water by suction and automatic washing devices of known type.

100 µl of ¹²⁵I-labeled SP-2 were put into each well. The plates were again incubated at 37°C for one hour. At the end of incubation, each small bead was transferred from a well to a tube by overturning the well. The radioactivity of each small bead was measured with a gamma counter.

The average value of the countings has been evaluated for each set of 3 test-tubes, after subtracting the value of the background. Standard curves were plotted on linear axes, representing the average cpm as the ordinates for each standard and the corresponding antigen 90K concentrations as the abscissas. The antigen 90K concentrations as U/ml were then read directly off the standard curve.

The threshold value for distinguishing the normal subjects from the pathological subjects was set forth at 11 U/ml. Therefore values higher than 11 U/ml are considered as positive.

The proportion of anti-HIV seronegative subjects at risk of HIV infection with elevated 90K antigen levels in the blood is higher (27%) with respect to the normal control population (5%). On the contrary, as shown in the table 1, 43% of the asymptomatic seropositive subjects, 71% of the subjects suffering from ARC and 94% of the subjects suffering from AIDS showed 90K antigen levels in the blood higher than the normal level.

**TABLE 1**

| 90K antigen in different classes of subjects | | | |
|---|---|---|---|
| | positive/total subjects | (%) | 90K U/ml |
| Control subjects | 9/170 | 5 | 5.5±3.3 |
| seronegative subjects exposed to the risk of AIDS | 35/130 | 27 | 11.8±14.5 |
| seropositive asymptomatic subjects | 47/108 | 43 | 14.9±6.9 |
| ARC | 40/56 | 71 | 22.2±14.3 |
| AIDS | 52/55 | 94 | 23.1±11.2 |

Such data point out that the evolution of the disease, passing through the asymptomatic anti-HIV seropositive stage, the ARC and evident AIDS stages, is associated with a progressive and significative increase of the 90K antigen level in the blood.

This has been also confirmed by the correlation observed between the 90K antigen levels in the blood and the sub-population of the CD4 lymphocytes, whose decrease in peripheral blood is a marker of the progression of the disease. More particularly, high levels of the 90K antigen in the blood turned out to be closely correlated (p less than 10⁻⁶) to a low CD4 countings. The advantage of 90K the antigen assay with respect to the counting of the lymphocytes CD4 consists in that the increase of the 90K antigen level precedes the start of the decrease in the CD4 lymphocytes. Indeed, when a remarkable decrease of the CD4 lymphocytes occurs, the damage to the immunosystem is irreversible by this time.

## Claims

1. Use of the SP-2 monoclonal antibody for an in vitro assay of the 90K antigen from biological fluids or tissues useful for the diagnostics related to HIV infection.

2. Use of the SP-2 monoclonal antibody according to claim 1 where said in vitro assay is performed to make a diagnosis of the disease stage during the HIV infection, to foresee the evolution of the infection through ARC or AIDS in anti-HIV seropositive asymptomatic subjects, to monitor the progress of HIV infection, to evaluate the efficacy of a specific (antiviral) or aspecific therapy, to early point out the serum conversion of anti-HIV seronegative subjects at risk.

3. Use of the SP-2 monoclonal antibody according to claim 2 where said in vitro assay is performed through immunoassays.

4. Use of the SP-2 monoclonal antibody according to claim 3 where said immunoassays comprise assays utilizing as revealing systems at least one of the following systems: enzymatic, radioisotopic or chemioluminescent.

5. Use of the SP-2 monoclonal antibody according to claim 4 where said enzymatic systems comprise ELISA or EIA assay and said radioisotopic systems comprise IRMA or RIA assays.

6. Use of the SP-2 monoclonal antibody according to any of the previous claims for the preparation of diagnostic kits to perform said in vitro assays of the 90K antigen.

## Patentansprüche

1. Benutzung von SP-2 monoklonalen Antikörpem für eine in vitro Probe des 90K Antigens von biologischen Flüssigkeiten oder Stoffen, die zur HIV Infektionen betreffenden Diagnostik nützlich ist.

2. Benutzung von SP-2 monoklonalen Antikörpem nach Anspruch 1, worin die vorgenannte in vitro Probe zur einer Diagnose des Krankheitsstandes während der HIV Infektion, zur Voraussicht der Entwicklung der Infektion durch ARC oder AIDS in seropositiven asymptomatischen Patienten, zur Überwachung des Fortschritts der HIV Infektion, zur Bewertung der Wikung einer spezifischen (antiviralen) oder aspezifischen Therapie und zur frühzeitigen Feststellung der Serumkonversion von anti-HIV seronegativen Risiko-Patienten, durchgeführt wird.

3. Benutzung von SP-2 monoklonalen Antikörpem nach Anspruch 2, worin die vorgenannte in vitro Probe durch Immunoproben durchgeführt wird.

4. Benutzung von SP-2 monoklonalen Antikörpern nach Anspruch 3, worin die vorgenannten Immunoproben Proben umfassen, die als Ermittlungssystem mindestens ein der folgenden Systeme anwenden: enzymatische, radioisotopische oder chemiolumineszente Systeme.

5. Benutzung von SP-2 monoklonalen Antikörpem nach Anspruch 4, worin die vorgenannten enzymatischen Systeme ELISA oder EIA Probe und die vorgenannten radioisotopischen Systeme IRMA oder RIA Proben umfassen.

6. Benutzung von SP-2 monoklonalen Antikörpem nach je einem der vorhergehenden Ansprüche zur Vorbereitung einer diagnostischen Ausrüstung für die Durchführung der vorgenannten in-vitro Proben des 90K Antigens.

## Revendications

1. Utilisation d'anticorps monoclonal SP-2 pour une preuve in vitro de l'antigène 90K des fluides ou tissus bilogiques utiles pour le diagnostic relatif à l'infection HIV.

2. Utilisation d'anticorps monoclonal SP-2 selon la revendication 1, dans laquelle ledit preuve in vitro est réalisé pour faire un diagnostic de degré de maladie pendant l'infection HIV, pour prévoir l'évolution de l'infection par ARC ou SIDA dans les sujets séropositves anti-HIV asymptomatiques, pour surveiller les progrés de l'infection HIV, pour évaluer l'efficacité d'un thérapie specifique (antiviral) ou aspécifique, pour mettre précocement en évidence la conversion de sérum des sujets négatives anti-HIV à risque.

3. Utilisation d'anticorps monoclonal SP-2 selon la revendication 2, dans laquelle ledit preuve in vitro est réalisé par des immunopreuves.

4. Utilisation d'anticorps monoclonal SP-2 selon la revendication 3, dans laquelle lesdits immunopreuves comprendant des preuves utilisant comme systèmes de détection au moins un des systèmes suivants: enzymatiques, radioisotopiques ou chimioluminescents.

5. Utilisation d'anticorps monoclonal SP-2 selon la revendication 4, dans laquelles lesdits systèmes enzymatiques comprendent les preuves ELISA ou EIA et lesdits systèms radioisotopiques comprendent les systèmes IRMA ou RIA

6. Utilisation d'anticorps monoclonal SP-2 selon une quelconque des revendications précédentes pour la préparation des kits diagnostiques pour réaliser lesdits preuves in vitro de l'antigene 90K.
